# EUROPEAN PATENT SPECIFICATION

(11) **EP 1 620 085 B1**
(45) Date of publication and mention of the grant of the patent: **23.07.2008**
(21) Application number: 04722967.9
(22) Date of filing: 24.03.2004
(51) Int. Cl.: A61K 31/216, A61K 31/195, A61K 31/192, A61K 31/191, A61P 13/10

(54) **USE OF PHENOXYACETIC ACID DERIVATIVES FOR TREATING HYPERACTIVE BLADDER**
VERWENDUNG VON PHENOXYESSIGSÄUREDERIVATEN ZUR BEHANDLUNG DER HYPERAKTIVEN BLASE
UTILISATION DE DERIVES DE L'ACIDE PHENOXYACETIQUE EN VUE DU TRAITEMENT D'UNE VESSIE HYPERACTIVE

(30) Priority: 05.05.2003 DE 10320084; 23.05.2003 DE 10323837
(43) Date of publication of application: 01.02.2006
(73) Proprietor: Kissei Pharmaceutical Co., Ltd., Matsumoto-shi Nagano 399-8710 (JP)
(72) Inventor: YAMAZAKI, Yoshinobu c/o Central Research Lab., Minamiazumi-gun, Nagano 399-8304 (JP); KOJIMA, Masami c/o Central Research Lab., Minamiazumi-gun, Nagano 399-8304 (JP)
(74) Representative: Hayes, Adrian Chetwynd
(86) International application number: PCT/JP2004/004048
(87) International publication number: WO 2004/098586

(56) References cited:
- EP-A- 1 095 932
- WO-A-03/024916

## Description

### Technical Field

The present invention relates to a new field of indication for phenoxyacetic acid derivatives according to European Patent Application EP 1095932. It has now been found that the compounds described therein are suitable for the preparation of a medicament for treating hyperactive bladder (Overactive Bladder: OAB). Accordingly, by means of these active substances, a method is provided for treating this urological syndrome.

### Background Art

The bladder function disorder OAB is a chronic widespread complaint which is estimated to affect more than 50 million people in the industrialised countries. According to the new terminology of the International Continence Society published in 2002, OAB is diagnosed symptomatically. The symptoms of OAB are imperative urinary urgency with or without urge incontinence, generally but not necessarily combined with pollacisuria and nycturia. OAB is also characterised by involuntary detrusor contractions which are either triggered by provocation or occur spontaneously. Two types of detrusor hyperactivity can be distinguished: if the detrusor hyperactivity observed is based on neurological causes (e.g. Parkinson's disease, apoplexy, some forms of multiple sclerosis or the cross section of the bone marrow) it is known as neurogenic detrusor hyperactivity. If no clear cause can be detected this is known as idiopathic detrusor hyperactivity. OAB has its own clinical picture which can be distinguished from other diseases with similar symptoms and should not be confused with diseases of this kind such as, for example, infections of the lower urinary tract, urothelial carcinoma, disorders of urine release, etc.

The less established methods of treatment include medicaments with antimuscarinics as the active substance. Some active substances from this category may be poorly tolerated or lead to a dry mouth on account of their poor selectivity for the urinary bladder. Side effects of this kind may constitute a limit to the therapy.

EP 1095932 discloses a number of phenoxyacetic acid derivatives from the catecholamine series. These compounds have a side chain resembling noradrenaline, except that not only the benzylic hydroxyl group but also the homobenzylic amino group is bound to an asymmetric carbon atom. The compounds described therein are credited with a positive effect in the treatment of urinary incontinence. The specification makes no comment as to the effect of these substances with regard to the treatment of overactive bladder.

It has now been found that these compounds are also suitable for treating the urological phenomenon of overactive bladder.

### Disclosure of the Invention

### 1. Description of the Invention

One aim of the present invention is to provide medicaments for treating overactive bladder.

As a further objective the present invention sets out to provide a new treatment option for treating overactive bladder.

The invention further sets out to discover new medical/pharmaceutical uses for phenoxyacetic acid derivatives from the catecholamine series.

A further aim of the invention is to improve the quality of life of people suffering from urological complaints, dysfunction or hyperactivity, particularly people with overactive bladder, using phenoxyacetic acid derivatives from the catecholamine series.

It is also an aim of the present invention to provide medicaments which specifically treat the corresponding physiological dysfunction without having unacceptable side effects which impair the quality of life of the patients affected.

### 2. Detailed Description of the Invention

The present invention relates to the use of phenoxyacetic acid derivatives according to EP 1095932 for preparing a medicament for the treatment of overactive bladder comprising as an active ingredient a phenoxyacetic acid derivative according to EP 1095932. According to EP 1095932 the compounds which form the basis for the use according to the invention are beta-3-adrenoceptor agonists. The substances may be used in particular for treating neurogenic bladder hyperactivity, neurogenic detrusor hyperactivity and also for treating idiopathic bladder hyperactivity and idiopathic detrusor hyperactivity.

The compounds which form the basis for the use according to the invention are represented by the following general formula I: 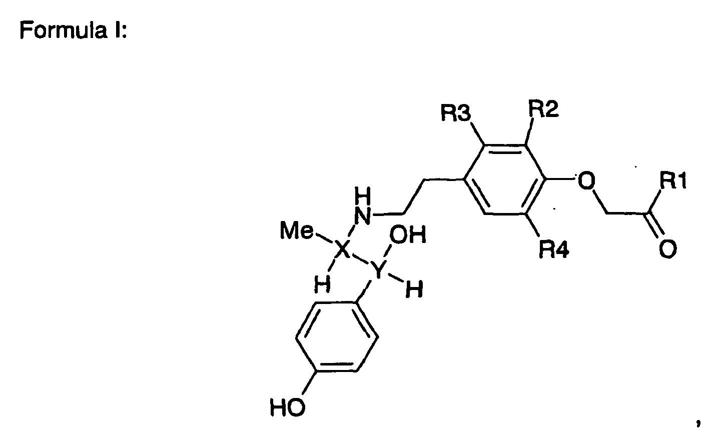 wherein
X is a chiral carbon atom of R or S, preferably S configuration,
Y is a chiral carbon atom of R or S, preferably R configuration,
the two stereocentres X and Y preferably being of opposite configurations, i.e. (R;S) or (S; R);
R1 is a hydroxy group, a C₁-C₆-alkoxy group, an aryl- C₁-C₆-alkoxy group, a primary amino group or a mono- or di (C₁-C₆-alkyl)amino group;
one of the groups R2 and R3 is a hydrogen atom, preferably R2, the other group is a hydrogen atom , a halogen atom, a C₁-C₆-alkyl group, a trifluoromethyl group or a C₁-C₆-alkoxy group;
R4 is a halogen atom, a C₁-C₆-alkyl group, a halo(C₁-C₆-alkyl) group, a hydroxy group, a C₁-C₆-alkoxy group, an aryl- C₁-C₆-alkoxy group, a C₁-C₆-alkoxy group, a cyano group, a nitro group, an amino group, a mono- or di (C₁-C₆-alkyl) amino group, a carbamoyl group, a mono- or di (C₁-C₆-alkyl)carbamoyl group or R4 corresponds to the group -NHCOR5, where R5 is a hydrogen atom or a C₁-C₆-alkyl group; or a pharmaceutically acceptable salt thereof.

In the present description of the invention the terms are defined as follows:
halogen atom: fluorine (F), chlorine (Cl), bromine (Br) or iodine (I);
C₁-C₆-alkyl: a branched or unbranched alkyl group with 1 to 6 carbon atoms, such as methyl, ethyl, propyl, isopropyl, butyl, isobutyl, sec.butyl, tert.butyl, pentyl, isopentyl, hexyl, etc.;
C₁-C₆-alkoxy: a branched or unbranched alkoxy group with 1 to 6 carbon atoms, methoxy, ethoxy, propoxy, isopropoxy, butoxy, isobutoxy, sec.butoxy, tert.butoxy, pentoxy, isopentoxy, hexoxy,etc.;
aryl: phenyl, naphthyl;
mono- or di (C₁-C₆-alkyl)amino group: denotes an amino group with one or two identical or different C₁-C₆-alkyl groups;
mono- or di (C₁-C₆-alkyl)carbamoyl group: denotes a carbamoyl group with one or two identical or different C₁-C₆-alkyl groups at the N function.

Methods of preparing the above-mentioned compounds are disclosed in EP 1095932. Analogous methods of preparation may be used for the synthesis of trifluoromethyl derivatives.

Preferred compounds are those of general formula I
wherein
X is a chiral carbon atom of S configuration,
Y is a chiral carbon atom of R configuration,
R1 is a hydroxy group, C₁-C₃-alkoxy group, an aryl- C₁-C₃-alkoxy group;
one of the groups R2 and R3 is a hydrogen atom, preferably R2, the other group is a C₁-C₃-alkyl group;
R4 is a C₁-C₃-alkyl group;
or a pharmaceutically acceptable salt thereof.

Within the scope of the present invention the compounds according to general formula II or pharmacologically acceptable salts thereof are particularly preferred. 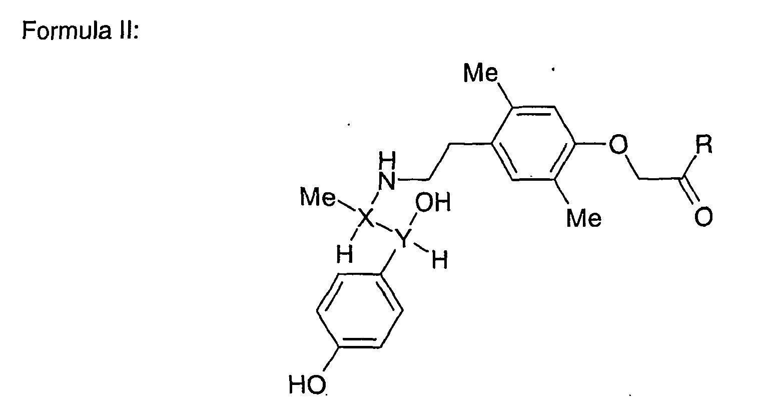 wherein
X is a chiral carbon atom of R or S, preferably S configuration,
Y is a chiral carbon atom of R or S, preferably R configuration,
the two stereocentres preferably being of opposite configurations, i.e. (R;S) or (S; R);
R denotes a hydroxy group, a methoxy or ethoxy group, preferably a hydroxy group or ethoxy group.

Most preferred are the compounds
● (-)-ethyl 2-[4-(2-{[(1S,2R)-2-hydroxy-2-(4-hydroxyphenyl)-1-methylethyl]amino}-ethyl)-2,5-dimethylphenoxy]acetate and
● (-)-2-[4-(2-{[(1S,2R)-2-hydroxy-2-(4-hydroxyphenyl)-1-methylethyl]amino}ethyl)-2,5-dimethylphenoxy]acetic acid.

The use according to the invention may be carried out with the neutral compounds and also with an acid addition salt or a solvate. Examples of such salts are those with inorganic acids, such as hydrochloric acid, hydrogen bromide, sulphuric acid, phosphoric acid or organic acids such as acetic acid, citric acid, tartaric acid, malic acid, succinic acid, fumaric acid, p-toluenesulphonic acid, benzenesulphonic acid, methanesulphonic acid, lactic acid, ascorbic acid, etc. The salts may be prepared from the neutral compounds by known methods.

In each case, the hydrochloride is the preferred salt form. In connection with this, WO 2003024916 may be mentioned, in particular, and reference is expressly made thereto. Of the salts mentioned above, the compound (-)-ethyl 2-[4-(2-{[(1S,2R)-2-hydroxy-2-(4-hydroxyphenyl)-1-methylethyl]amino}ethyl)-2,5-dimethylphenoxy]-acetate hydrochloride described in WO 2003024916 is particularly preferred within the scope of the present invention.

The compounds characterised by formula I or II are part of a pharmaceutical medicament, according to the invention.

According to the invention, the complaint of overactive bladder is to be treated by the administration of one of the compounds, pharmaceutical formulations or medicaments according to the invention.

The medication according to the invention may be given orally, by inhalation, by intravenous or transdermal route or as a suppository. Oral administration is preferred.

To determine the optimum dose of the active substance for use and formulation according to the invention, various parameters must be taken into consideration, such as the patient's age and body weight and the nature and stage of the disease, for example.

The preferred dose for humans is between 0.001 mg and 1 g per day, preferably between 10 mg and 500 mg.

In some cases a smaller amount may be sufficient while in other cases a larger overall amount may be necessary.

The overall daily dose may be taken as a single dose or in several batches over the day depending on the treatment programme. The treatment programme may also prescribe intervals of more than one day between the doses.

For oral administration, various pharmaceutical formulations are available such as solids, liquids, powders, tablets, sugar-coated tablets, capsules, coated tablets, granules, suspensions, solutions, syrups, sublingual tablets or other forms.

A powder may be prepared for example by grinding the particles of active substance to a suitable size. Diluted powders may be produced by finely grinding the powdered active substance with a non-toxic carrier such as lactose and forming a powder therefrom. Other suitable carrier materials are other carbohydrates such as starch or mannitol. These powders may optionally contain flavours, preservatives, dispersing agents, colourings and other pharmacological excipients.

Capsules may be prepared starting from a powder of the kind mentioned above or other powders which are enclosed in a capsule, preferably a gelatine capsule, after which the capsule is sealed.

It is also possible to introduce lubricants known from the prior art into the capsule or use them to seal the two capsule sections. The effectiveness of a capsule when taken orally can be increased by the addition of disintegrants or solubilisers such as carboxymethylcellulose, carboxymethylcellulose calcium, low-substituted hydroxy propylcellulose, calcium carbonate, sodium carbonate and other substances. The active substance may be present in the calcium not only as a solid but also in suspension, e.g. in vegetable oil, polyethyleneglycol, glycerol, using surface-active substances, etc.

Tablets may be produced by compressing the powdered mixture and then processing it to form granules, for example. The tablets may contain various excipients such as starches, lactose, sucrose, glucose (e.g. for vaginal tablets), sodium chloride, urea for tablets for dissolving or injecting, amylose, different types of cellulose as described above and so on. Glycerol or starch may be used as a moisture retaining agent, for example.

Starch, alginic acid, calcium alginate, pectic acid, powdered agar-agar, formaldehyde gelatine, calcium carbonate, sodium bicarbonate, magnesium peroxide and amylose may be used as disintegrants, for example.

As anti-disintegrants or solution retardants it is possible to use, for example, sucrose, stearine, solid paraffin (preferably with a melting point in the range from 50-52°C); cocoa butter and hydrogenated fats.

Suitable absorption accelerators include, *inter alia,* quaternary ammonium compounds, sodium lauryl sulphate and saponins.

Ether may be used as the binder distributor, for example, while the hydrophilisation agent or breakdown accelerator used may be cetylalcohol, glycerol monostearate, starch, lactose and wetting agents (e.g. aerosol OT, Pluronics, Tweens) and the like.

The following may also be generally used as additional excipients: ⁺Aerosil, Aerosol OT ethylcellulose, Amberlite resin, XE-88, Amijel, Amisterol, amylose, Avicel microcrystalline-cellulose, bentonite, calcium sulphate, Carbowax 4000 and 6000, carrageen, castor wax, cellulose, microcrystalline cellulose, dextrane, dextrin, pharmaceutical tablet base, kaolin, spray dried lactose (USP), lactosil, magnesium stearate, mannitol, granular mannitol N. F. methylcellulose, Miglyol 812 neutral oil, powdered milk, lactose, nal-tab, nepol-amylose, Pöfizer crystalline sorbitol, plasdone, polyethyleneglycols, polyvinylpyrrolidone, Précirol, neat's foot oil (hydrogenated), melting tablet base, silicone, stabiline, Sta-rx 1500, syloid, Waldhof tablet base, tablettol, talcum cetylatum and stearatum, Tego metal soaps, fructose and tylose. The tabletting excipient K (M25) is particularly suitable, and also complies with the requirements of the following pharmacopoeias: DAB, Ph, Eur, BP and NF.

Other excipients known from the prior art may also be used.

The tablets may be produced by direct compression, for example.
It is also possible to prepare other formulations for oral administration such as solutions, syrups, elixirs etc. If desired the compound may be micro-encapsulated.

Parenteral administration may be achieved by dissolving the compound in a liquid and injecting it by subcutaneous, intramuscular or intravenous route. Suitable solvents include, for example, water or oily media.

In order to prepare suppositories, e.g. vaginal pessaries, the compound may be formulated with low-melting and water-soluble or water-insoluble materials such as polyethylene glycol, cocoa butter, higher esters (for example moerysthyl, palmitate) or mixtures thereof.

To prepare transdermal formulations, ointments, creams or plasters may be used.

### Brief Description of Drawings

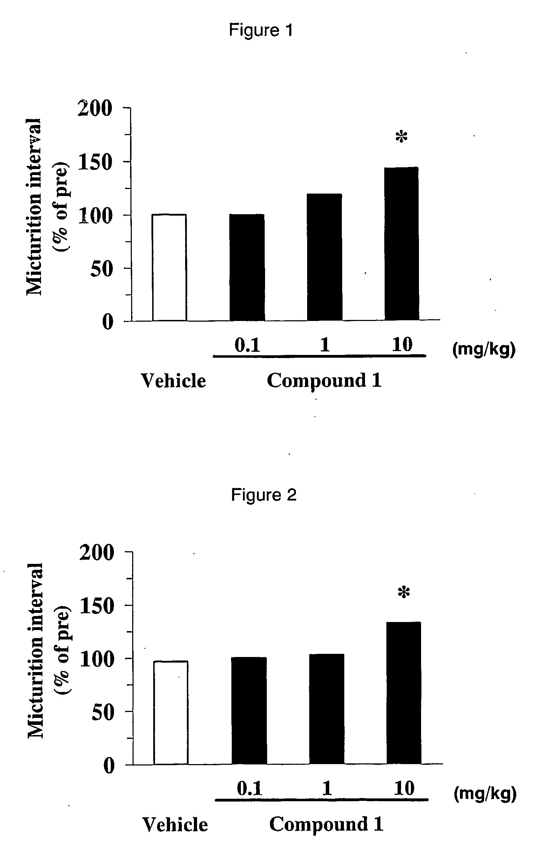

Figure 1 is a graph illustrating effects of intragastric administration of ethyl (-)-2-[4-(2-{[(1S,2R)-2-hydroxy-2-(4-hydroxyphenyl)-1-methylethyl]amino}ethyl)-2,5-dimethylphenoxy] acetate hydrochloride (Compound 1) on the micturition interval in conscious rat with PGE₂-induced bladder hyperactivity at 1 hour after administration. The axis of ordinates shows micturition interval (% of pre), and the axis of abscissas shows sorts and doses (mg/kg) of drugs. The symbol * in the graph shows the significant difference from the vehicle group at p<0.05.
Figure 2 is a graph illustrating effects of intragastric administration of Compound 1 on the micturition interval in conscious rat with PGE₂-induced bladder hyperactivity at 2 hours after administration. The axis of ordinates shows micturition interval (% of pre), and the axis of abscissas shows sorts and doses (mg/kg) of drugs. The symbol * in the graph shows the significant difference from the vehicle group at p<0.05.

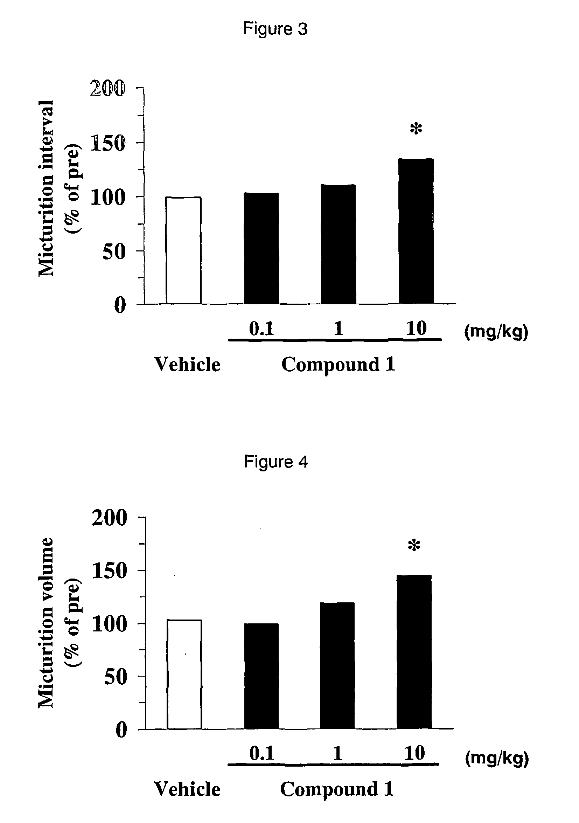

Figure 3 is a graph illustrating effects of intragastric administration of Compound 1 on the micturition interval in conscious rat with PGE₂-induced bladder hyperactivity at 4 hours after administration. The axis of ordinates shows micturition interval (% of pre), and the axis of abscissas shows sorts and doses (mg/kg) of drugs. The symbol * in the graph shows the significant difference from the vehicle group at p<0.05.
Figure 4 is a graph illustrating effects of intragastric administration of Compound 1 on the micturition volume in conscious rat with PGE₂-induced bladder hyperactivity at 1 hour after administration. The axis of ordinates shows micturition volume (% of pre), and the axis of abscissas shows sorts and doses (mg/kg) of drugs. The symbol * in the graph shows the significant difference from the vehicle group at p<0.05.

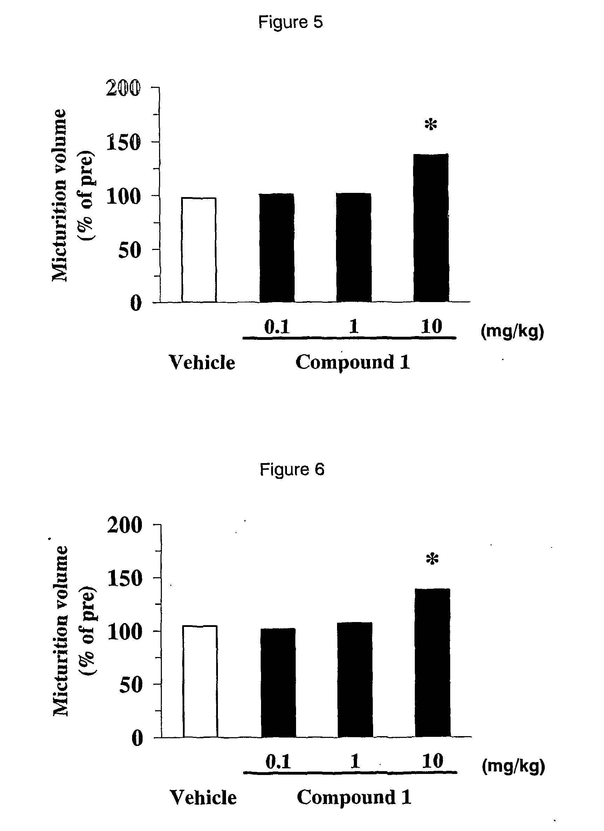

Figure 5 is a graph illustrating effects of intragastric administration of Compound 1 on the micturition volume in conscious rat with PGE₂-induced bladder hyperactivity at 2 hours after administration. The axis of ordinates shows micturition volume (% of pre), and the axis of abscissas shows sorts and doses (mg/kg) of drugs. The symbol * in the graph shows the significant difference from the vehicle group at p<0.05.
Figure 6 is a graph illustrating effects of intragastric administration of Compound 1 on the micturition volume in conscious rat with PGE₂-induced bladder hyperactivity at 4 hours after administration. The axis of ordinates shows micturition volume (% of pre), and the axis of abscissas shows sorts and doses (mg/kg) of drugs. The symbol * in the graph shows the significant difference from the vehicle group at p<0.05.

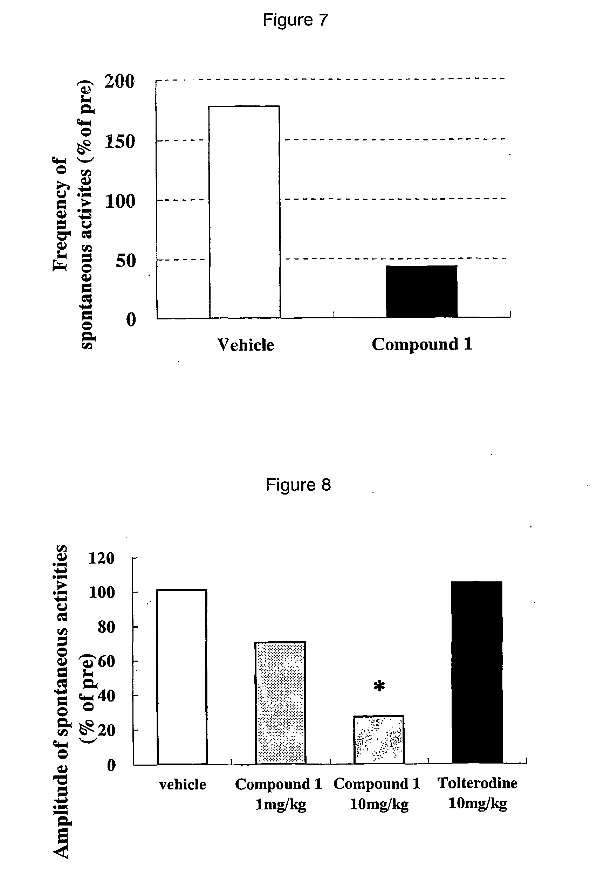

Figure 7 is a graph illustrating effects of intragastric administration of Compound 1 on the frequency of spontaneous small bladder constraction in filling phase in spinal cord injury-induced overactive bladder model. The axis of ordinates shows frequency of spontaneous activities (% of pre), and the axis of abscissas shows sorts of drugs.
Figure 8 is a graph illustrating effects of intragastric administration of Compound 1 on the amplitude of spontaneous small bladder contraction in filling phase in lower urinary tract partially obstructed overactive bladder model. The axis of ordinates shows amplitude of spontaneous activities (% of pre), and the axis of abscissas shows sorts and doses (mg/kg) of drugs. The symbol * in the graph shows the significant difference from the vehicle group at p<0.05.

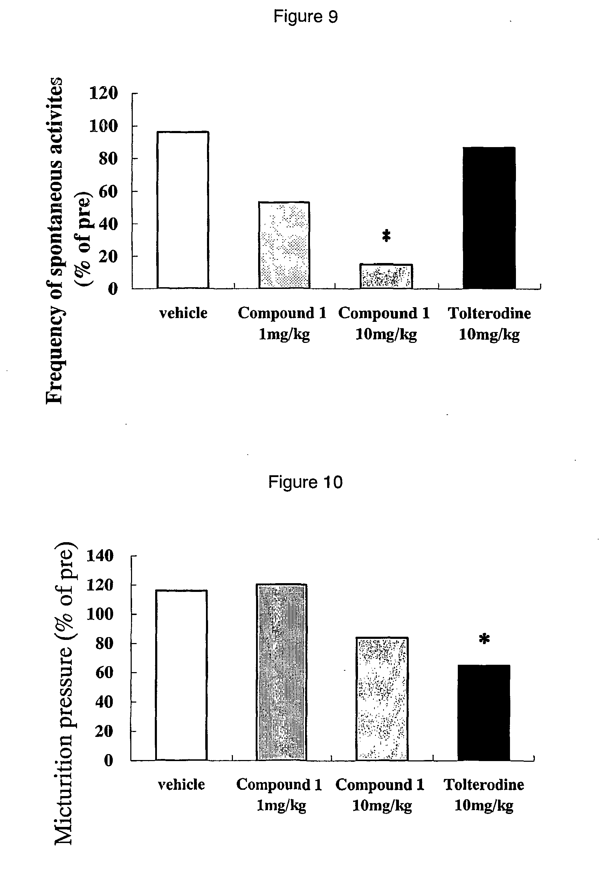

Figure 9 is a graph illustrating effects of intragastric administration of Compound 1 on the frequency of spontaneous small bladder contraction in filling phase in lower urinary tract partially obstructed overactive bladder model. The axis of ordinates shows frequency of spontaneous activities (% of pre), and the axis of abscissas shows sorts and doses (mg/kg) of drugs. The symbol * in the graph shows the significant difference from the vehicle group at p<0.05.
Figure 10 is a graph illustrating effects of intragastric administration of Compound 1 on the micturition pressure of spontaneous small bladder contraction in filling phase in lower urinary tract partially obstructed overactive bladder model. The axis of ordinates shows micturition pressure (% of pre), and the axis of abscissas shows sorts and doses (mg/kg) of drugs. The symbol ∗ in the graph shows the significant difference from the vehicle group at p<0.05.

### Best Mode for Carrying Out the Invention

On the effect of (-)-2-[4-(2-{[(1S,2R)-2-hydroxy-2-(4-hydroxyphenyl)-1-methylethyl]-amino}ethyl)-2,5-dimethylphenoxy] acetic acid, the active metabolite of (-)-ethyl 2-[4-(2-{[(1S,2R)-2-hydroxy-2-(4-hydroxyphenyl)-1-methylethyl]amino}ethyl)-2,5-dimethylphenoxy]acetate, a new beta-3-agonist on the isolated monkey detrusor and the rat bladder.

The present experiments demonstrate the effect of (-)-2-[4-(2-{[(1S,2R)-2-hydroxy-2-(4-hydroxyphenyl)-1-methylethyl]amino}ethyl)-2,5-dimethylphenoxy] acetic acid on the isolated monkey detrusor and the rat bladder.

### Example 1

### Effects on the tone of the isolated monkey detrusor

### Method

The detrusor of the cynomolgus monkey (both sexes) was isolated and dissected. Tracheal, atrial and urethral dissections were also prepared. Then the effect of (-)-2-[4-(2-{[(1S,2R)-2-hydroxy-2-(4-hydroxyphenyl)-1-methylethyl]amino}ethyl)-2,5-dimethylphenoxy] acetic acid on the tone of the detrusor preparation was tested. Carbachol-induced tonic contractions of the tracheal preparations, the heart rate of the atrial preparations and endothelin-1-induced tonic contractions of the urethral preparations were also investigated using the Magnus method.

### Results

Both (-)-2-[4-(2-{[(1S,2R)-2-hydroxy-2-(4-hydroxyphenyl)-1-methylethyl]amino}ethyl)-2,5-dimethylphenoxy] acetic acid and also isoproterenol reduce the tone of the isolated monkey detrusor. The EC₅₀ values of the two substances were 8.2× 10⁻⁷ M, and 1.9 × 10⁻⁷ M, respectively. No significant relaxation was observed using the two anti-muscarinic active substances propiverine or oxybutynin. Isoproterenol reduced the carbachol-induced tonic contraction of the isolated trachea (beta 2-AR-stimulated function) and increased the heart rate of the isolated atria (beta1-AR- stimulated function), in concentration-dependent manner in each case. (-)-2-[4-(2-{[(1S,2R)-2-Hydroxy-2-(4-hydroxyphenyl)-1-methylethyl]amino}ethyl)-2,5-dimethylphenoxy] acetic acid exhibited less effect on the trachea and atria. The detrusor selectivity of (-)-2-[4-(2-{[(1 S,2R)-2-hydroxy-2-(4-hydroxyphenyl)-1-methylethyl]amino}ethyl)-2,5-dimethylphenoxy] acetic acid was about 1200 times greater (compared with the trachea) and 80 times greater (compared with the atria). (-)-2-[4-(2-{[(1S,2R)-2-Hydroxy-2-(4-hydroxyphenyl)-1-methylethyl]amino}ethyl)-2,5-dimethylphenoxy]acetic acid showed no effect on the endothelin-1-induced tonic contraction of the isolated urethra.

### Example 2

### Effects in prostaglandin (PG) E₂-induced rat overactive bladder model

### Method

Rats were anesthetized with pentobarbital sodium. Each cannula was implanted into the urinary bladder and stomach, tunneled subcutaneously and secured on the back of the neck and closed. Seven days after the cannula implantation, cystometrogram of the freely-moved conscious rats were measured. Saline was continuously instilled into the bladder at a rate of 6 mL/hour, and after the cystometric parameters were stabilized, PGE₂ (60 µmol/L)-containing saline was instilled continuously instead of the saline into the bladder. After the stable shortening of the micturition interval was confirmed under the condition, ethyl (-)-2-[4-(2-{[(1 S,2R)-2-hydroxy-2-(4-hydroxyphenyl)-1-methylethyl]amino}ethyl)-2,5-dimethylphenoxy] acetate hydrochloride (0 (vehicle), 0.1, or 10 mg/kg as doses of the free body) was administered intragastrically. Cystometric parameters were measured for 4 hours after the administration and were expressed as a percentage to those at the pre-administration (0 hour).

### Results

Intra-bladder instillation of PGE₂ caused a shortening of the micturition interval and decrease of micturion volume. Ethyl (-)-2-[4-(2-{[(1S,2R)-2-hydroxy-2-(4-hydroxyphenyl)-1-methylethyl]amino}ethyl)-2,5-dimethylphenoxy] acetate hydrochloride prolonged the micturition interval and increased the micturition volume dose-dependently in the PGE₂-induced rat overactive bladder model (Figures 1 to 6).

### Example 3

### Effects in spinal cord injury-induced overactive bladder model

### Method

In ether anesthetized rats, spinal transactions were performed at the level of Th9-Th10. Each rat was treated with amikacin 10 mg/body, i.m. for 7 days, and micturition management was done 2 times/day for 2 weeks to prevent over-distension of the bladder. About 6 weeks after the spinal cord operation, rats were anesthetized with pentobarbital sodium and each cannula (PE-50: Nihon Becton Dickinson) was implanted into the urinary bladder and stomach, tunneled subcutaneously, secured on the back of the neck and closed. Seven days after the cannula implantation, cystometrogram was performed. Saline was instilled into the urinary bladder at a rate of 12 mL/hour. The saline instillation was stopped every micturition. After the stable spontaneous small bladder contraction in filling phase was confirmed, ethyl (-)-2-[4-(2-{[(1S,2R)-2-hydroxy-2-(4-hydroxyphenyl)-1-methylethyl]amino}ethyl)-2,5-dimethylphenoxy] acetate hydrochloride (0 (vehicle) or 10 mg/kg as dose of free body) was administered intragastrically. The micturition parameter at 1 hour after the administration of ethyl (-)-2-[4-(2-{[(1 S,2R)-2-hydroxy-2-(4-hydroxyphenyl)-1-methylethyl]amino}ethyl)-2,5-dimethylphenoxy] acetate hydrochloride was measured and was expressed as a percentage to that at the pre-administration (0 hour).

### Results

The spontaneous small bladder contractions in filling phase were appeared in spinal cord injured rats. Ethyl (-)-2-[4-(2-{[(1S,2R)-2-hydroxy-2-(4-hydroxyphenyl)-1-methylethyl]amino}ethyl)-2,5-dimethylphenoxy] acetate hydrochloride decreased the frequency of the spontaneous small bladder contraction in filling phase in this spinal cord injury-induced overactive bladder model (Figure 7).

### Example 4

### Effects in lower urinary tract partially obstructed rat overactive bladder model

### Method

Rats were anesthetized with pentobarbital sodium. After urethra was ligated with a tube (width: 1 mm) by thread, the tube was removed. Six weeks after the lower urinary tract operation, the implanted thread was removed, and each cannula was implanted into the urinary bladder and stomach, tunneled subcutaneously and secured on the back of the neck and closed. The next day, cystometrogram of freely-moved conscious rats were measured. Saline was instilled into the urinary bladder at a rate of 12 mL/hour. The saline instillation was stopped every micturition. After the stable spontaneous small bladder contraction in filling phase was confirmed, ethyl (-)-2-[4-(2-{[(1S,2R)-2-hydroxy-2-(4-hydroxyphenyl)-1-methylethyl]amino}ethyl)-2,5-dimethylphenoxy] acetate hydrochloride (0 (vehicle), 1 or 10 mg/kg as doses of free body) or tolterodine (10 mg/kg) was administered intragastrically. The micturition parameters at 1 hour after the administration of ethyl (-)-2-[4-(2-{[(1S,2R)-2-hydroxy-2-(4-hydroxyphenyl)-1-methylethyl]amino}ethyl)-2,5-dimethylphenoxy] acetate hydrochloride or tolterodine were measured and were expressed as a percentage to that at the pre-administration (0 hour).

### Results

Spontaneous small bladder contraction in filling phase was observed in rats 6 weeks after the partial obstruction of the lower urinary tract. Ethyl (-)-2-[4-(2-{[(1 S,2R)-2-hydroxy-2-(4-hydroxyphenyl)-1-methylethyl]amino}ethyl)-2,5-dimethylphenoxy] acetate hydrochloride administered intragastrically decreased the amplitude and frequency of the spontaneous small bladder contractions dose-dependently. The potency of ethyl (-)-2-[4-(2-{[(1S,2R)-2-hydroxy-2-(4-hydroxyphenyl)-1-methylethyl]-amino}ethyl)-2,5-dimethylphenoxy] acetate hydrochloride was stronger than that of tolterodine, an anti-muscarinic drug (Figures 8 and 9). On the other hand, ethyl (-)-2-[4-(2-{[(1 S,2R)-2-hydroxy-2-(4-hydroxyphenyl)-1-methylethyl]amino}ethyl)-2,5-dimethylphenoxy] acetate hydrochloride did not affect the micturition pressure (Figure 10). These results showed that ethyl (-)-2-[4-(2-{[(1 S,2R)-2-hydroxy-2-(4-hydroxyphenyl)-1-methylethyl]amino}ethyl)-2,5-dimethylphenoxy] acetate hydrochloride inhibits the spontaneous small bladder contraction in filling phase with not affecting the micturition pressure, and suggested that the compound is useful for prevention and treatment of the overactive bladder.

### Conclusion

(-)-2-[4-(2-{[(1S,2R)-2-Hydroxy-2-(4-hydroxyphenyl)-1-methylethyl]amino}ethyl)-2,5-dimethylphenoxy]acetic acid exhibited detrusor selectivity. In addition, ethyl (-)-2-[4-(2-{[(1S,2R)-2-hydroxy-2-(4-hydroxyphenyl)-1-methylethyl]amino}ethyl)-2,5-dimethylphenoxy] acetate hydrochloride prolonged the micturition interval, increased the micturition volume, and decreased the amplitude and frequency of the spontaneous small bladder contractions dose-dependently when administered intragastrically. These show that (-)-ethyl 2-[4-(2-{[(1S,2R)-2-hydroxy-2-(4-hydroxyphenyl)-1-methylethyl]amino}ethyl)-2,5-dimethylphenoxy]acetate can be used as a "prodrug" of (-)-2-[4-(2-{[(1S,2R)-2-hydroxy-2-(4-hydroxyphenyl)-1-methylethyl]-amino}ethyl)-2,5-dimethylphenoxy]acetic acid as a therapeutic agent for the treatment of overactive bladder and has fewer side effects than the active substances known from the prior art.

### Industrial Applicability

As described above, the compounds represented by the above general formula (I) and pharmaceutically acceptable salts thereof are extremely useful for treating overactive bladder.

## Claims

1. A use of a compound of general formula 1 for preparing a medicament for the treatment of overactive bladder, 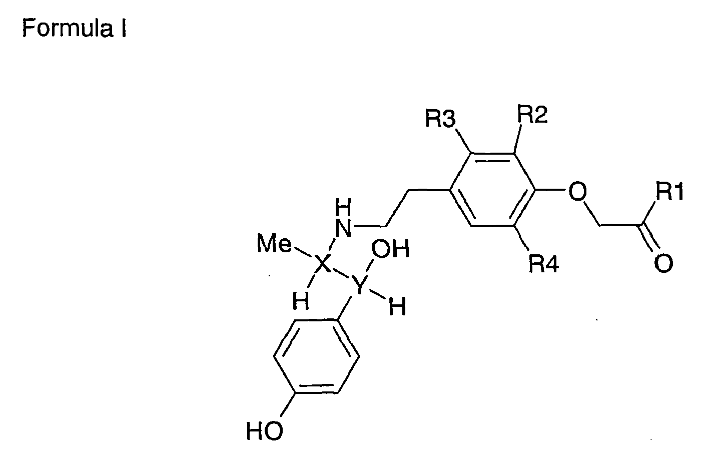 wherein
X is a chiral carbon atom of R or S;
Y is a chiral carbon atom of R or S;
R1 is a hydroxy group, a C₁-C₆-alkoxy group, an aryl- C₁-C₆-alkoxy group, a primary amino group or a mono- or di (C₁-C₆-alkyl)amino group;
one of the groups R2 and R3 is a hydrogen atom, the other group is a hydrogen atom , a halogen atom, a C₁-C₆-alkyl group, a trifluoromethyl group or a C₁-C₆-alkoxy group; and
R4 is a halogen atom, a C₁-C₆-alkyl group, a halo(C₁-C₆-alkyl) group, a hydroxy group, a C₁-C₆-alkoxy group, an aryl- C₁-C₆-alkoxy group, a C₁-C₆-alkoxy group, a cyano group, a nitro group, an amino group, a mono- or di (C₁-C₆-alkyl)amino group, a carbamoyl group, a mono- or di (C₁-C₆-alkyl)carbamoyl group or corresponds to the group -NHCOR5, where R5 is a hydrogen atom or a C₁-C₆-alkyl group;
or a pharmaceutically acceptable salt thereof.

2. A use according to claim 1, **characterised in that** the two stereocentres X and Y are of opposite configuration.

3. A use according to claim 2, **characterised in that** the stereocentre X on which the amino group is formed is of S configuration and the stereocentre Y on which the hydroxy group is formed is of R configuration.

4. A use according to one of claims 1 to 3, **characterised in that**
R1 is a hydroxy group, a C₁-C₃-alkoxy group or an aryl- C₁-C₃-alkoxy group;
one of the groups R2 and R3 is a hydrogen atom, the other group is a C₁-C₃-alkyl group; and
R4 is a C₁-C₃-alkyl group;
or a pharmaceutically acceptable salt thereof.

5. A use according to claim 4, **characterised in that**
R1 is a hydroxy group, a methoxy group or an ethoxy group;
R2 is a hydrogen atom;
R3 is a methyl group; and
R4 is a methyl group;
or a pharmaceutically acceptable salt thereof.

6. A use according to claim 5, **characterised in that**
R1 is a hydroxy group or an ethoxy group;
or a pharmaceutically acceptable salt thereof.

7. A use according to one of claims 1 to 6, **characterised in that** the compound is a pharmaceutically acceptable salt with one of the acids selected from among hydrochloric acid, hydrogen bromide, sulphuric acid, phosphoric acid, acetic acid, citric acid, tartaric acid, malic acid, succinic acid, fumaric acid, p-toluenesulphonic acid, benzenesulphonic acid, methanesulphonic acid, lactic acid or ascorbic acid.

8. A use according to claim 1, **characterised in that** the compound is (-)-ethyl 2-[4-(2-{[(1S,2R)-2-hydroxy-2-(4-hydroxyphenyl)-1-methylethyl]amino}ethyl)-2,5-dimethylphenoxy]acetate, (-)-ethyl 2-[4-(2-{[(1S,2R)-2-hydroxy-2-(4-hydrooyphenyl)-1-methylethyl]amino}ethyl)-2,5-dimethylphenoxy]acetate hydrochloride or (-)-2-[4-(2-{[(1S,2R)-2-hydroxy-2-(4-hydroxyphenyl)-1-methylethyl]amino}ethyl)-2,5-dimethylphenoxy] acetic acid.

9. A use according to one of claims 1 to 8, **characterised in that** the medicament is an oral preparation.

10. A use according to one of claims 1 to 8, **characterised in that** the medicament is a suppository.

11. A use according to one of claims 1 to 8, **characterised in that** the medicament is a transdermal plaster.

12. A use according to one of claims 1 to 11, for preparing a medicament for treating neurogenic bladder hyperactivity.

13. A use according to one of claims 1 to 11, for preparing a medicament for treating Idiopathic bladder hyperactivity.

## Patentansprüche

1. Verwendung einer Verbindung mit der allgemeinen Formel 1 zur Herstellung eines Medikaments zur Behandlung der hyperaktiven Blase, 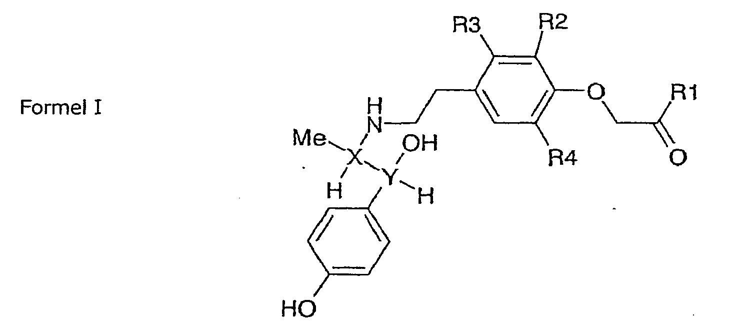 worin:
X ein chirales Kohlenstoffatom von R oder S; und
Y ein chirales Kohlenstoffatom von R oder S ist;
R1 eine Hydroxygruppe, eine C₁-C₆-Alkoxygruppe, eine Aryl-C₁-C₆-Alkoxygruppe, eine primäre Aminogruppe oder eine Mono- oder Di(C₁-C₆-Alkyl)aminogruppe ist;
eine der Gruppen R2 und R3 ein Wasserstoffatom, und die andere Gruppe ein Wasserstoffatom, ein Halogenatom, eine C₁-C₆-Alkylgruppe, eine Trifluormethylgruppe oder eine C₁-C₆-Alkoxygruppe ist; und
R4 ein Halogenatom, eine C₁-C₆-Alkylgruppe, eine Halo(C₁-C₆-Alkyl)gruppe, eine Hydroxygruppe, eine C₁-C₆-Alkoxygruppe, eine Aryl-C₁-C₆-Alkoxygruppe, eine C₁-C₆-Alkoxygruppe, eine Cyanogruppe, eine Nitrogruppe, eine Aminogruppe, eine Mono- oder Di(C₁-C₆-Alkyl)aminogruppe, eine Carbamoylgruppe, eine Mono- oder Di(C₁-C₆-Alkyl)carbamoylgruppe ist oder der Gruppe -NHCOR5 entspricht, wobei R5 ein Wasserstoffatom oder eine C₁-C₆-Alkylgruppe ist;
oder ein pharmazeutisch verträgliches Salz davon.

2. Verwendung nach Anspruch 1, **dadurch gekennzeichnet, dass** die beiden Stereozentren X und Y eine gegensätzliche Konfiguration aufweisen.

3. Verwendung nach Anspruch 2, **dadurch gekennzeichnet, dass** das Stereozentrum X, auf dem die Aminogruppe gebildet wird, eine S-Konfiguration aufweist, und das Stereozentrum Y, auf dem die Hydroxygruppe gebildet wird, eine R-Konfiguration aufweist.

4. Verwendung nach einem der Ansprüche 1 bis 3, **dadurch gekennzeichnet, dass**
R1 eine Hydroxygruppe, eine C₁-C₃-Alkoxygruppe oder eine Aryl-C₁-C₃-Alkoxygruppe ist; eine der Gruppen R2 und R3 ein Wasserstoffatom, die andere Gruppe eine C₁-C₃-Alkylgruppe ist; und
R4 eine C₁-C₃-Alkylgruppe ist;
oder ein pharmazeutisch verträgliches Salz davon.

5. Verwendung nach Anspruch 4, **dadurch gekennzeichnet, dass**
R1 eine Hydroxygruppe, eine Methoxygruppe oder eine Ethoxygruppe;
R2 ein Wasserstoffatom;
R3 eine Methylgruppe; und
R4 eine Methylgruppe ist;
oder ein pharmazeutisch verträgliches Salz davon.

6. Verwendung nach Anspruch 5, **dadurch gekennzeichnet, dass**
R1 eine Hydroxygruppe oder eine Ethoxygruppe ist;
oder ein pharmazeutisch verträgliches Salz davon.

7. Verwendung nach einem der Ansprüche 1 bis 6, **dadurch gekennzeichnet, dass** die Verbindung ein pharmazeutisch veträgliches Salz ist, wobei eine der Säuren aus Salzsäure, Bromwasserstoff, Schwefelsäure, Phosphorsäure, Essigsäure, Citronensäure, Weinsäure, Äpfelsäure, Bernsteinsäure, Fumarsäure, p-Toluolsulfonsäure, Benzolsulfonsäure, Methansulfonsäure, Milchsäure oder Ascorbinsäure ausgewählt ist.

8. Verwendung nach Anspruch 1, **dadurch gekennzeichnet, dass** die Verbindung (-)-Ethyl 2-[4-(2-{[(1S,2R)-2-hydroxy-2-(4-hydroxyphenyl)-1-methylethyl]amino}ethyl)-2,5-dimethylphenoxy]acetat, (-)-Ethyl 2-[4-(2-{[(1S,2R)-2-hydroxy-2-(4-hydroxyphenyl)-1-methylethyl]amino}ethyl)-2,5-dimethylphenoxy]acetathydrochlorid oder (-)-Ethyl 2-[4-(2-{[(1S,2R)-2-hydroxy-2-(4-hydroxyphenyl)-1-methylethyl]amino}ethyl)-2,5-dimethylphenoxy]essigsäure ist.

9. Verwendung nach einem der Ansprüche 1 bis 8, **dadurch gekennzeichnet, dass** das Medikament eine orale Zubereitung ist.

10. Verwendung nach einem der Ansprüche 1 bis 8, **dadurch gekennzeichnet, dass** das Medikament ein Suppositorium ist.

11. Verwendung nach einem der Ansprüche 1 bis 8, **dadurch gekennzeichnet, dass** das Medikament ein transdermales Pflaster ist.

12. Verwendung nach einem der Ansprüche 1 bis 11 zur Herstellung eines Medikaments zur Behandlung der neurogenen hyperaktiven Blase.

13. Verwendung nach einem der Ansprüche 1 bis 11 zur Herstellung eines Medikaments zur Behandlung der idiopathischen hyperaktiven Blase.

## Revendications

1. Utilisation d'un composé de formule générale 1 pour la préparation d'un médicament destiné au traitement de l'hyperactivité vésicale, 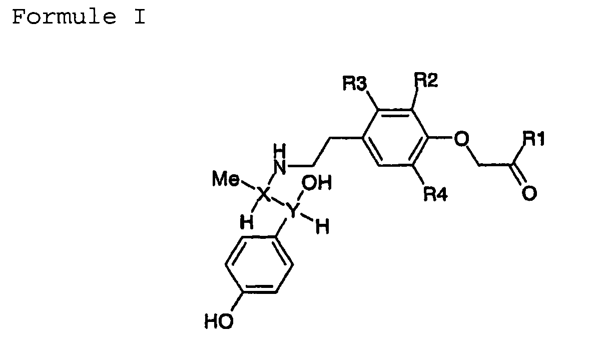 dans laquelle
X représente un atome de carbone chiral R ou S ;
Y représente un atome de carbone chiral R ou S ;
R1 représente un groupe hydroxy, un groupe alkoxy en C₁ à C₆ , un groupe aryl-(alkoxy en C₁ à C₆), un groupe amino primaire ou un groupe mono- ou di(alkyle en C₁ à C₆) amino ;
un des groupes R2 et R3 représente un atome d'hydrogène, l'autre groupe représente un atome d'hydrogène, un atome d'halogène, un groupe alkyle en C₁ à C₆, un groupe trifluorométhyle ou un groupe alkoxy en C₁ à C₆ ; et
R4 représente un atome d'halogène, un groupe alkyle en C₁ à C₆, un groupe halogénalkyle en C₁ à C₆, un groupe hydroxy, un groupe alkoxy en C₁ à C₆, un groupe aryl-(alkoxy en C₁ à C₆), un groupe alkoxy en C₁ à C₆, un groupe cyano, un groupe nitro, un groupe amino, un groupe mono- ou di (alkyle en C₁ à C₆) amino, un groupe carbamoyle, un groupe mono- ou di(alkyle en C₁ à C₆)carbamoyle, ou bien correspond au groupe -NHCOR5, dans lequel R5 représente un atome d'hydrogène ou un groupe alkyle en C₁ à C₆ ;
ou d'un de ses sels pharmaceutiquement acceptables.

2. Utilisation suivant la revendication 1, **caractérisée en ce que** les deux stéréocentres X et Y ont la configuration opposée.

3. Utilisation suivant la revendication 2, **caractérisée en ce que** le stéréocentre X, sur lequel le groupe amino est formé, a la configuration S et le stéréocentre Y, sur lequel le groupe hydroxy est formé, a la configuration R.

4. Utilisation suivant l'une des revendications 1 à 3, **caractérisée en ce que**
R1 représente un groupe hydroxy, un groupe alkoxy en C₁ à C₃ ou un groupe aryl-(alkoxy en C₁ à C₃);
l'un des groupes R2 et R3 représente un atome d'hydrogène, l'autre groupe représente un groupe alkyle en C₁ à C₃ ; et
R4 représente un groupe alkyle en C₁ à C₃ ;
ou d'un de ses sels pharmaceutiquement acceptables.

5. Utilisation suivant la revendication 4, **caractérisée en ce que**
R1 représente un groupe hydroxy, un groupe méthoxy ou un groupe éthoxy ;
R2 représente un atome d'hydrogène;
R3 représente un groupe méthyle ; et
R4 représente un groupe méthyle ;
ou d'un de ses sels pharmaceutiquement acceptables

6. Utilisation suivant la revendication 5, **caractérisée en ce que**
R1 représente un groupe hydroxy ou un groupe éthoxy ;
ou d'un de ses sels pharmaceutiquement acceptables.

7. Utilisation suivant l'une des revendications 1 à 6, **caractérisée en ce que** le composé est un sel pharmaceutiquement acceptable formé avec un des acides choisis entre l'acide chlorhydrique, le bromure d'hydrogène, l'acide sulfurique, l'acide phosphorique, l'acide acétique, l'acide citrique, l'acide tartrique, l'acide malique, l'acide succinique, l'acide fumarique, l'acide p-toluènesulfonique, l'acide benzènesulfonique, l'acide méthanesulfonique, l'acide lactique et l'acide ascorbique.

8. Utilisation suivant la revendication 1, **caractérisée en ce que** le composé est le 2-[4-(2-{[(1S,2R)-2-hydroxy-2-(4-hydroxyphényl)-1-méthyléthyl]amino}éthyl)-2,5-diméthylphénoxy]acétate de (-) -éthyle, le chlorhydrate de 2-[4-(2-{[(1S,2R)-2-hydroxy-2-(4-hydroxyphényl)-1-méthyléthyl]amino}éthyl)-2,5-diméthylphénoxy]acétate de (-)-éthyle ou l'acide (-)-2-[4-(2-{[(1S,2R)-2-hydroxy-2-(4-hydroxyphényl)-1-méthyléthyl] - amino}éthyl)-2,5-diméthylphénoxy]acétique.

9. Utilisation suivant l'une des revendications 1 à 8, **caractérisée en ce que** le médicament est une préparation orale.

10. Utilisation suivant l'une des revendications 1 à 8, **caractérisée en ce que** le médicament est un suppositoire.

11. Utilisation suivant l'une des revendications 1 à 8, **caractérisée en ce que** le médicament est un emplâtre transdermique.

12. Utilisation suivant l'une des revendications 1 à 11, pour la préparation d'un médicament destiné au traitement d'une hyperactivité vésicale neurogène.

13. Utilisation suivant l'une des revendications 1 à 11, pour la préparation d'un médicament destiné au traitement d'une hyperactivité vésicale idiopathique.
